Europäisches Patentamt

European Patent Office

Office européen des brevets

(19)

(11) Publication number: 0 319 091
A2

# EUROPEAN PATENT APPLICATION

(21) Application number: 88202697.4

(22) Date of filing: 28.11.88

(51) Int. Cl.⁴: C07H 13/06

(30) Priority: 04.12.87 GB 8728385
18.12.87 GB 8729563

(43) Date of publication of application:
07.06.89 Bulletin 89/23

(84) Designated Contracting States:
AT BE CH DE ES FR GB GR IT LI NL SE

(71) Applicant: UNILEVER NV
Burgemeester s'Jacobplein 1 P.O. Box 760
NL-3000 DK Rotterdam(NL)

(84) BE CH DE ES FR GR IT LI NL SE AT

Applicant: UNILEVER PLC
Unilever House Blackfriars P.O. Box 68
London EC4P 4BQ(GB)

(84) GB

(72) Inventor: van Lookeren, Gerard Johannes
Hjortevej 27
DK-6400 Sonderborg(DK)

(74) Representative: Mulder, Cornelis Willem
Reinier, Dr. et al
UNILEVER N.V. Patent Division P.O. Box 137
NL-3130 AC Vlaardingen(NL)

(54) Method of purifying crude polyol fatty acid polyesters.

(57) The present invention pertains to a method of purifying crude polyol fatty acid polyesters synthesized and/or purified by a process involving contact with alkali metal ions under alkaline conditions, comprising the step of reducing the level of alkali metal ions to less than 5 ppm by weight. The invention further pertains to polyesters having such reduced residual levels of alkali metal ions, and to food compositions in particular for frying purposes, comprising such polyesters as fat-replacers.

EP 0 319 091 A2

EP 0 319 091 A2

# METHOD OF PURIFYING CRUDE POLYOL FATTY ACID POLYESTERS

The present invention relates to a method of purifying crude polyol fatty acid polyesters, and in particular, although not exclusively, crude sugar fatty acid polyesters. The present invention further relates to polyol fatty acid polyesters thus produced or producable, and to food products containing them.

Polyol fatty acid polyesters and in particular, the sugar fatty acid polyesters such as e.g. the sucrose fatty acid polyesters, are known as suitable low-calorie fat-replacers in edible products. Substantially indigestible for human beings they have physical and organoleptic properties very similar to triglyceride oils and fats conventionally used in edible products. In addition, polyol fatty acid polyesters are reported to have use as pharmaceutical agents e.g. in view of their ability to take up fat-soluble substances such as in particular cholesterol, in the gastro-intestinal tract, and subsequently remove these substances from the human body.

In this specification the term "polyol" is intended to include any aliphatic or aromatic compound which comprises at least four free hydroxyl groups. Such polyols in particular include the group of sugar polyols, which comprises the sugars, i.e. the mono-, di- and polysaccharides. the corresponding sugar alcohols and the derivatives thereof having at least four free hydroxyl groups. Examples of sugar polyols include glucose, mannose, galactose, xylose, fructose, sorbose, tagatose, ribulose, xylulose, maltose, lactose, cello-biose, raffinose, sucrose, erythritol, mannitol, lactitol, sorbitol, xylitol and $\alpha$-methylglucoside. A generally used sugar polyol is sucrose.

The term "polyol fatty acid polyester" is intended to include any such polyesters or mixtures thereof of which on an average, more than 70 % of the polyol hydroxyl groups have been esterified with fatty acids.

The term "fatty acid" refers to $C_8$-$C_{24}$ fatty acids which may be saturated or unsaturated, and may have straight or branched alkyl chains.

In general polyol fatty acid polyesters are synthesized by a process in which a polyol, such as a mono- or disaccharide, is reacted with a fatty acid lower alkylester, in general the fatty acid methylester, in the presence of a transesterification catalyst. such as e.g. an alkali metal hydroxide or carbonate. In a first stage a polyol fatty acid mono- or oligoester is formed, which in a second stage is further reacted with the fatty acid lower alkylester to form polyesters of the desired degree of esterification. It is also possible to combine the two stages of the reaction into a single step.

Processes of this type are described in e.g. the US patent specifications Nos. 3,963,699, 4,517.360, and 4,518,772.

The crude polyol fatty acid polyester reaction products resulting from conventional syntheses contain in addition to the desired polyesters, components such as fatty acid soaps, excess fatty acid lower alkylesters and polyol fatty acid oligoesters. Also, due to the relatively high temperatures at which conventional processes are carried out, often by-products are formed which may be undesirable in view of their chemical characteristics, such as in particular discolouring properties. In general it is therefore necessary to further purify or refine the crude polyol fatty acid polyester reaction products resulting from such conventional syntheses.

For the purposes of this specification crude polyol fatty acid polyesters are defined as unrefined or incompletely refined reaction products of processes for the synthesis of polyol fatty acid polyesters. Such crude compositions in general contain of from 10 to 95 % by weight of polyol fatty acid polyesters.

Conventional purification methods include washing with water, extraction with organic solvents, and/or salting-out treatments. In the US patent specification No. 4,334,061 there is described a process for the preparation of sucrose polyesters, in which the reaction product is washed using an aqueous alkaline solution of pH 7-12 in the presence of a polar organic solvent.

Such conventional washing methods are not always fully reliable. In particular, if a full refining process is used including e.g. conventional steam-stripping or deodorizing steps, discolouring of the polyesters may again occur, even though initially an almost de-coloured product resulted after the washing step.

It has now been found that this tendency to discolour at subsequent high temperature treatments or uses, such as deodorizing in a full refining procedure or frying in the case the polyol fatty acid polyesters are incorporated in food products intended for frying purposes, is influenced by the level of alkali metal ions in the polyester product subjected to the high temperature treatment. In particular, it has been found that the level of alkali metal ions which are introduced under or to achieve alkaline conditions during synthesis (basic catalyst) or alkaline washing treatments should be reduced to under 5 ppm calculated by weight of the polyol fatty acid polyester. It is believed that even very low amounts of by-products resulting from contact with alkali metal ions under alkaline conditions give rise to discolouring tendency under subsequent

2

high-temperature conditions.

Accordingly, in a first aspect the present invention provides a method of purifying crude polyol fatty acid polyesters synthesized and/or purified by a process involving contact with alkali metal ions under alkaline conditions, comprising the step of reducing the level of alkali metal ions to less than 5 ppm calculated by weight of said polyesters.

In a second aspect the present invention provides polyol fatty acid polyesters synthesized and/or purified by a process involving contact with alkali metal ions under alkaline conditions said polyesters comprising a residual level of said alkali metal ions of less than 5 ppm calculated by weight of said polyesters.

In a further aspect the present invention provides food compositions containing fat material in which at least part of the digestible fat material is replaced by polyol fatty acid polyesters synthesized and/or refined by a process involving contact with alkali metal ions under alakline conditions, said polyesters comprising a residual level of said alkali metal ions of less than 5 ppm calculated by weight of said polyesters.

The present invention is applicable to methods of synthesis and/or purification of polyol fatty acid polyesters involving the use of 'alkaline' alkali metal ions, i.e. alkali metal ions which have been in contact with the polyesters or the preceding oligoesters at some point during the synthesis or purification process. Such contact during synthesis may be due to the use of alkaline transesterification catalysts, such as alkali metal hydrides, hydroxides, alkoxides, and/or carbonates. Contact during purification may be due to washing steps with aqueous alkaline solutions, optionally in combination with organic solvents and/or electrolytes, such as sodium chloride.

An essential feature of the present invention is that the level of such alkali metal ions should be reduced to below 5 ppm calculated by weight of the polyol fatty acid polyesters.

Although an exact relationship between residual level of alkaline alkali metal ions and the degree of discolouring that may occur at later high-temperature treatments or uses, is not known, this relationship is believed to be monotonous, and accordingly, the lower the residual level the less likely it is that discolouring may occur. Preferably therefore, residual levels are reduced to below 1 ppm, reduction to residual levels of below 0.5 ppm have been found to give best results.

Suitable reductions to the low residual alkali metal levels in accordance with the present invention may be achieved by suitable sequences of washing treatments and/or bleaching steps.

A suitable method of purifying crude polyol fatty acid polyester products involves an initial alkaline washing step in which the polyol fatty acid polyester product is submitted to a treatment with a suitable amount of an aqueous alkaline solution having a pH of at least 12.5.

Somewhat dependent of the desired or necessary degree of purification and of the further purification or refining steps that may be employed, a suitable amount of the aqueous alkaline solution varies between 0.1 to 15 % by weight of the crude polyol fatty acid polyesters. In particular, amounts in the range of from 2 to 12 % by weight, preferably of from 5 to 10 % by weight, have been found suitable.

Conventional alkaline materials can be used to provide aqueous alkaline solutions having a pH of at least 12.5. Suitable such materials include alkali metal and earth alkali metal hydroxides. Preferably, sodium hydroxide is used. The aqueous alkaline solution preferably has a pH of at least 13 corresponding to concentrations of the alkaline material of at least 0.1 N. Preferably, 1 to 9 N NaOH solutions are used corresponding to aqueous alkaline solutions of pH 14 and higher. Most preferably, aqueous NaOH solutions having a strength of 2 to 6 N are applied.

The alkaline washing step is preferably carried out at elevated temperature, in particular at a temperature of from 40 to 110° C, and most preferably at a temperature of from 60 to 100° C.

In general the crude polyesters and the aqueous alkaline solution are agitated to ensure sufficient contact between the components of the polyester mixture and the washing solution. In batch-wise operation contacting times of less than one hour are normally sufficient. In particular, contacting times lie within the range of 1 to 30 minutes, 3 to 15 minutes being preferred. In a continuous operation, e.g. where the aqueous alkaline solution is in-line dosed to the crude polyester and the mixture is subsequently centrifuged, contact times in general are less than about 3 minutes, in particular, less than about 1 minute, and can be as short as 5 to 30 seconds.

Purification results may be further advantageously affected when a sequence of two or more washing steps with aqueous alkaline solutions.

Dependent of the amount of soap present in the crude polyesters it may be of further advantage to have the alkaline washing step be preceded by a reduction of the soap level. Suitably, such a reduction of the soap level is effectuated by way of centrifuging or filtrating off crystallised soap, and/or one or more preceding washing treatments with aqueous near-neutral solutions having a pH in the range of 4-9, in particular of 5-8. The aqueous near-neutral solution may further advantageously comprise an electrolyte, such as NaCl, in an amount of up to 2 %, preferably in the rage of from 0.3-1.5 % by weight of the crude polyesters.

In general the aqueous near-neutral washing solution is used in an amount of up to 50 % by weight of the crude polyesters. Amounts of up to 15 %, particularly, in the range of from 5 to 10 % by weight are preferred.

If a sequence of two or more alkaline washing steps is applied, also the first alkaline washing step can be suitably used to achieve said initial reduction of the soap level.

Although the polyester phase and the water phase in the alkaline and the optional near-neutral washing steps can be separated by gravity settling only, separation by centrifuging is preferred.

Subsequently, the refining process may and preferably does include a further acid washing step in which the polyol fatty acid polyesters resulting after the alkaline washing step are treated with an aqueous solution of an acid such as in particular phosphoric acid or citric acid. The alkaline and the optional acid washing steps are followed by further washing treatments with aqueous near-neutral solutions, and/or drying steps.

Subsequent to the washing treatments the polyol fatty acid polyesters may be and preferably are further treated using conventional refining techniques such as bleaching with activated carbon and/or bleaching earth, distilling at a temperature of about 170 to 210°C, and/or deodorizing, such as steam-stripping, at a temperature of 180 to 260°C, preferably 190 to 220°C.

It will be clear that before the high temperature treatments such as distilling and deodorizing the reduction of the residual level of alkali metal ions in accordance with the invention must have taken place.

Although the method according to the invention is suitable for purifying crude products of the general group of polyol fatty acid polyesters as defined hereinbefore, it is particularly suitable for purifying crude products comprising polyol fatty acid polyesters of which, on an average, more than 80 %, or even 90 % of the hydroxyl groups of the polyol have been esterified with fatty acids. In particular, such polyesters derived from the sugar polyols selected from the group of disaccharides or the alcohol derivatives thereof, such as sucrose, and esterified to over 95 % fatty acid substitution, are suitably purified by the method in accordance with the present invention.

Methods in accordance with the invention results in polyol fatty acid polyesters comprising a residual level of alkali metal ions of less than 5 ppm, in particular less than 1 ppm and most preferably less than 0.5 ppm.

Having a reduced risk of discolouring such polyesters are particularly suitable to replace fully or partially conventional triglyceride fats in food compositions intended for high-temperature purposes, such as baking and frying oils. Generally, in such food compositions at least 10 % by weight of the conventional triglyceride fat is replaced by the polyol fatty acid polyesters in accordance with the present invention. Preferably, at least 50 % of the conventional fat is replaced by the polyesters.

The invention is now further illustrated with reference to the following examples, percentages being by weight unless indicated otherwise

## EXAMPLE 1

A crude sucrose fatty acid polyester mixture (polyester fatty acid residues derived from non-distilled soybean methyl esters) comprising about 40 % of fatty acid methylester and about 10 % of soap was heated up to 80°C in a stirred vessel, 10 % of an aqueous 10 % NaCl solution was added and after 15 minutes the soap phase was settled with a centrifuge. The sucrose fatty acid polyester / fatty acid methylester-phase was washed twice with 5 % of 4 N NaOH solution at 80°C. The soap content at this stage was 400 ppm. To remove the residual soap 2 % of citric acid was added and after strong agitation the sucrose fatty acid polyester / fatty acid methylester mixture was washed pH neutral with water and dried. By measuring the soap content with atomic adsorption spectroscopy analyzing for sodium and potassium, it was found that the levels of both alkali metals were below 0.1 ppm.

The colour in the Lovibond $5\frac{1}{4}$ " cell after drying was 30 Yellow + 3.0 Red. After bleaching and deodorizing at 190°C the final colour was 30 Yellow + 3.2 Red in the $5\frac{1}{4}$ " cell.

## EXAMPLE 2

A crude sucrose fatty acid polyester (the polyester fatty acid residues derived from non-distilled soybean methylesters) was refined using a refining procedure similar to example 1 resulting in colour characteristics after bleaching and deodorizing as indicated in Table 1. The level of Na and K after bleaching was measured to be below 0.25 ppm.

To investigate in a comparative way the influence of 'alkaline' alkali metal ions on the colour properties of refined polyesters the above refined batch of sucrose polyester was deodorised at 220°C and 1 mbar for 30 minutes with the following additions: (1) 10 ppm $Na^+$ as NaCl, (2) 10 ppm $Na^+$ as $Na_2CO_3$. (3) 1 ppm $Na^+$ as NaOH, (4) 1 ppm $Na^+$ as NaOH plus 10 ppm $H^+$ as HCl, and (5) 10 ppm $H^+$ as HCl. After the deodorizing the colour was measured using a 1" Lovibond cell. The results in Table 1 clearly indicated the adverse effect on colour stability by the presence of low amounts of alkali metal ions in their 'alkaline' form.

TABLE 1

| Test materials | Lovibond colour - 1" cell | |
| --- | --- | --- |
| | Yellow | Red |
| sucrose polyester (no added ions) before test deodorizing | 20 | 1.1 |
| sucrose polyester (no added ions) after test deodorizing | 20 | 1.5 |
| sucrose polyester + 10 ppm $Na^+$ as NaCl after test deodorizing | 23 | 2.0 |
| sucrose polyester + 10 ppm $Na^+$ as $Na_2CO_3$ after test deodorizing | > 60 | > 6 |
| sucrose polyester + 1 ppm $Na^+$ as NaOH after test deodorizing | 60 | 5.1 |
| sucrose polyester + 1ppm $Na^+$ as NaOH plus 10 ppm $H^+$ as HCl after test deodorizing | 30 | 3.2 |
| sucrose polyester + 10 ppm $H^+$ as HCl after test deodorizing | 20 | 2.0 |

## Claims

1. A method of purifying crude polyol fatty acid polyesters synthesized and/or purified by a process involving contact with alkali metal ions under alkaline conditions, characterised in that it comprises the step of reducing the level of alkali metal ions to less than 5 ppm calculated by weight of said polyesters.

2. A method according to claim 1 which comprises the step of reducing the level of alkali metal ions to less than 1 ppm calculated by weight of said polyesters.

3. A method according to claim 2 which comprises the step of reducing the level of alkali metal ions to less than 0.5 ppm calculated by weight of said polyesters.

4. Polyol fatty acid polyesters purified by a method according to any one of the preceding claims.

5. Food compositions containing fat material in which at least part of the digestible fat material is replaced by polyol fatty acid polyesters according to claim 4.

6. Food compositions according to claim 5 which is a frying oil.